# EUROPEAN PATENT APPLICATION

(11) **EP 1 595 952 A1**
(43) Date of publication of application: **16.11.2005**
(21) Application number: 04709740.7
(22) Date of filing: 10.02.2004
(51) Int. Cl.: C12N 15/10

(54) **METHOD OF ESTIMATING ANTITUMOR EFFECT OF HISTONE DEACETYLASE INHIBITOR**

(30) Priority: 19.02.2003 JP 2003041790
(71) Applicant: Astellas Pharma Inc., Tokyo 103-8411 (JP)
(72) Inventor: SASAKAWA, Yuka, c/o Astellas Pharma Inc., Tokyo 103-8411 (JP); NAOE, Yoshinori, Yokohama-shi, Kanagawa 245-0016 (JP)
(74) Representative: Gille Hrabal Struck Neidlein Prop Roos
(86) International application number: PCT/JP2004/001419
(87) International publication number: WO 2004/074478

(57) **Abstract**

The present invention provides a method of obtaining a gene capable of becoming an index for predicting the efficacy of a histone deacetylase inhibitor, which comprises at least
(I) a step of dividing tumor cells into a histone deacetylase inhibitor sensitive tumor cell and a histone deacetylase inhibitor resistant tumor cell, and
(II) a step of examining the gene expression pattern of each of the sensitive tumor cell and the resistant tumor cell, and a step of selecting
   (i) a gene showing high expression in the sensitive tumor cell and low expression in the resistant tumor cell, or
   (ii) a gene showing low expression in the sensitive tumor cell and high expression in the resistant tumor cell, in order to provide a gene useful for predicting the efficacy, particularly an antitumor effect, of a histone deacetylase inhibitor, for a tumor desired to be treated.

## Description

### Technical Field

The present invention relates to a method of obtaining a gene useful for predicting an efficacy, specifically an antitumor effect, of a histone deacetylase inhibitor, and a method of predicting the efficacy (antitumor effect) of the histone deacetylase inhibitor, which comprises examining an expression pattern of the gene.

### Background Art

In recent years, a "tailor made medicine" is gaining recognition, which takes into consideration individual differences between patients, and a search for a marker to distinguish a cancer against which a pharmaceutical agent is effective from a cancer against which the pharmaceutical agent is ineffective is considered to be necessary. It is an attempt to ethically and medically improve cost performance of medication treatment by administering a pharmaceutical agent to patients after verification in advance of the probability of effect thereof, thereby to enhance efficacy as well as avoid toxicity of the pharmaceutical agent, and to reduce insignificant use of the pharmaceutical agent. In cancer treatment, the development of a method for predicting the efficacy of anticancer agents has been desired, because it can be an important means to bridge the gap between basic study and clinical application.

For example, it has been reported that a compound represented by the formula (I) 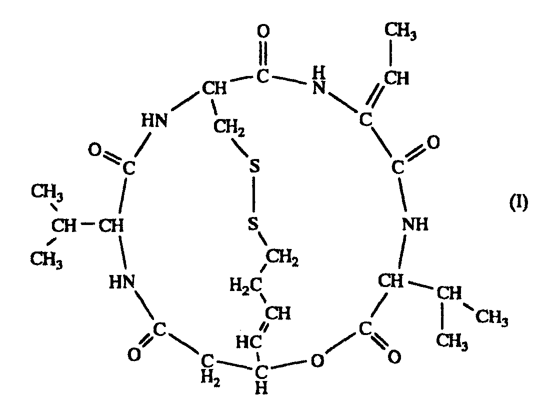 (hereinafter to be also referred to as compound A; SEQ ID NO: 1), particularly a stereoisomer (FK228) represented by the formula (II) 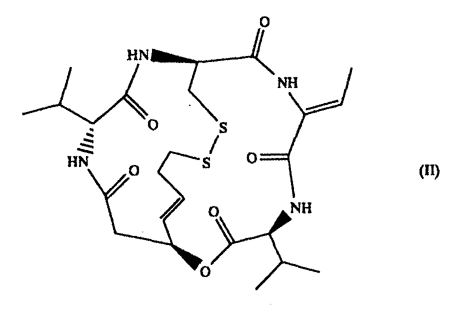 has a histone deacetylase inhibitory action and induces a potent antitumor activity (e.g., JP-B-7-64872, Experimental Cell Research, US (1998), vol. 241, pp. 126-133). However, no report has established a factor capable of predicting an antitumor effect of this compound, and as the situation stands, many problems are yet to be solved, such as whether or not in vitro results directly apply *in vivo,* whether or not the compound shows a practical effect *in vivo* in any tumor and the like.

### Disclosure of the Invention

An object of the present invention is to provide a method of obtaining a gene capable of becoming an index of efficacy prediction, which is useful for predicting the efficacy of a histone deacetylase inhibitor, particularly compound A known to have a potent histone deacetylase inhibitory action, and a method of predicting the efficacy of the histone deacetylase inhibitor, which comprises examining the expression pattern of the gene.

The present inventors have conducted intensive studies in an attempt to solve the above-mentioned problems and found that the antitumor effect of the histone deacetylase inhibitor varies depending on the kind of tumor, and the variation is observed in association with changes in the expression pattern of a particular gene or protein. They have identified these particular genes (proteins), observed the expression pattern of these genes in tumor, and based on such observation, found a method of predicting or evaluating the efficacy of a histone deacetylase inhibitor, which resulted in the completion of the present invention. Accordingly, the present invention provides the following.
(1) A method of obtaining a gene capable of becoming an index for predicting efficacy of a histone deacetylase inhibitor, which comprises at least
   (I) a step of examining an in vivo antitumor effect of a histone deacetylase inhibitor for tumor cells, and dividing the tumor cells into a cell type sensitive to the histone deacetylase inhibitor (sensitive tumor cell) and a cell type resistant thereto (resistant tumor cell), and
   (II) a step of examining the gene expression pattern of each of the sensitive tumor cell and the resistant tumor cell divided in the above-mentioned step (I), and selecting
      (i) a gene showing high expression in the sensitive tumor cell and low expression in the resistant tumor cell, or
      (ii) a gene showing low expression in the sensitive tumor cell and high expression in the resistant tumor cell.
(2) The method of (1), wherein the histone deacetylase inhibitor is a compound represented by the following chemical formula (I): 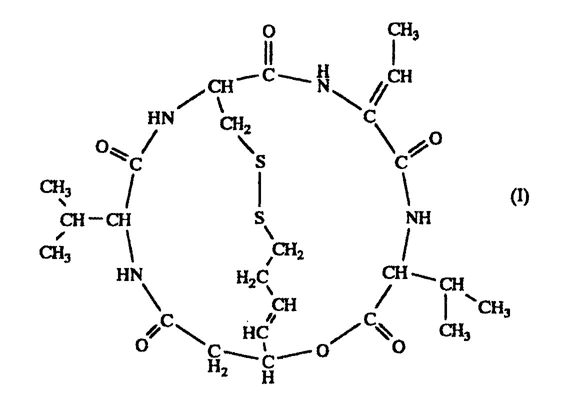 or a pharmaceutically acceptable salt thereof.
(3) The method of (1) or (2), wherein the tumor cell is derived from prostate cancer, stomach cancer or kidney cancer.
(4) A method of predicting an antitumor effect of a histone deacetylase inhibitor, which comprises examining an expression amount of at least one gene obtained by the method of (1) in at least one kind of tumor cell.
(5) The method of (4), wherein the histone deacetylase inhibitor is a compound represented by the following chemical formula(I): 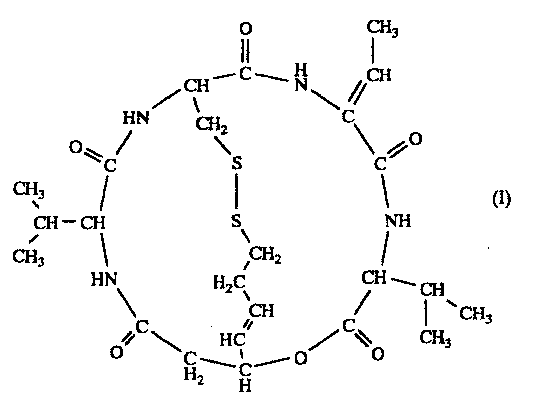 or a pharmaceutically acceptable salt thereof.
(6) The method of (4) or (5), wherein the at least one gene shows high expression in a sensitive cell and low expression in a resistant tumor cell.
(7) The method of (4) or (5), wherein the at least one gene shows low expression in a sensitive cell and high expression in a resistant tumor cell.
(8) A method of predicting an antitumor effect of a histone deacetylase inhibitor, which comprises examining an expression amount of at least one gene showing high expression in a sensitive cell and low expression in a resistant tumor cell, and at least one gene showing low expression in a sensitive cell and high expression in a resistant tumor cell, which are obtained by the method described in (1), in at least one kind of tumor cell.
(9) The method of any of (4)-(8), wherein the tumor cell is derived from prostate cancer, stomach cancer or kidney cancer.

### Brief Description of the Drawings

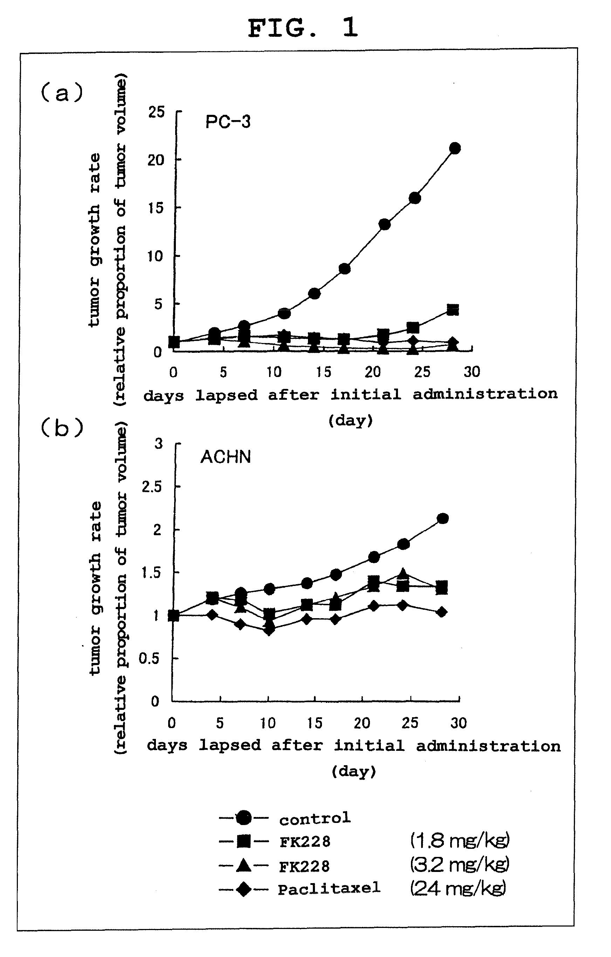

Fig. 1 is a graph showing an antitumor effect of FK228 on human prostate cancer ((a); PC-3) and kidney cancer ((b); ACHN), wherein the vertical axis shows a tumor growth rate, the transverse axis shows the number of days lapsed from the initial administration, and the tumor growth rate is expressed in a relative ratio of tumor volume after day 0 relative to that on day 0 as 1.

### Detailed Description of the Invention

The present invention provides a method of obtaining a gene capable of becoming an index for predicting an efficacy, particularly an antitumor effect, of a histone deacetylase inhibitor. By analyzing the expression pattern of the gene obtained by the method in a tumor cell, the information such as whether the histone deacetylase inhibitor is useful for the treatment, or whether the histone deacetylase inhibitor is effective for the target cancer and the like can be obtained, which makes it possible to contribute to the "tailor made medicine". In addition, a histone deacetylase inhibitor capable of exhibiting an antitumor effect in a target tumor cell can be found without actual administration to the human body.

The "histone deacetylase inhibitor" means a compound that binds to an active site of histone deacetylase competitively with a substrate, or a compound having an action to bind with a site different from the active site of histone deacetylase to change the enzyme activity of the histone deacetylase, and includes compounds whose use as a histone deacetylase inhibitor is known, all compounds (synthetic and natural) whose histone deacetylase inhibitory activity has been reported and all the compounds that will be reported in the future. Specifically, the aforementioned compound A, a salt thereof, and a derivative thereof (e.g., acetylated compound A, thiol form with reduced S-S bond etc.) can be mentioned. Moreover, tricostatin A, sodium butyrate, suberoylanilide hydroxamic acid (SAHA), MS-275, Cyclic hydroxamic-acid-containing peptide, Apicidin, Trapoxin and the like are the compounds whose histone deacetylase inhibitory activity has been reported.

While compound A may have a steric isomer, such as optical isomer, geometric isomer and the like, which is based on the asymmetric carbon atom and a double bond (e.g., FK228), all of these isomers and mixtures thereof are also encompassed in the histone deacetylase inhibitor to be used in the present invention.

Furthermore, solvate compounds of compound A, FK228 or a salt thereof (e.g., inclusion compound (e.g., hydrate etc.)) are also encompassed in the scope of the invention.

In the present specification, unless otherwise specified, a simple reference to compound A means a compound group irrespective of stereoisomerism, including the compound represented by the formula (II) (FK228).

The compound A and a salt thereof are known substances and available. For example, FK228, which is one of the stereoisomers of compound A, can be obtained by culturing a strain belonging to the genus Chromobacterium, which is capable of producing FK228 under aerobic conditions, and harvesting the substance from the culture broth. As the strain belonging to the genus Chromobacterium, which is capable of producing FK228, for example, Chromobacterium violaceum WB968 (FERM BP-1968) can be mentioned. FK228 can be more specifically obtained from the producing strain as described in JP-B-7-64872 (US Patent No. 4977138). FK228 is preferably harvested from the strain belonging to the genus Chromobacterium, which is capable of producing FK228, because FK228 can be more easily obtained, and synthetic and semisynthetic FK228 are also advantageous because further purification step is not necessary or can be simple. Similarly, compound A other than FK228 can be semi-synthesized or totally synthesized by a conventionally known method. More specifically, it can be produced according to the method reported by Khan W. Li, et al. (J. Am. Chem. Soc., vol. 118, 7237-7238 (1996)).

As a salt of compound A, salts with inorganic base (e.g., alkali metal salts such as sodium salt, potassium salt and the like, alkaline earth metal salts such as calcium salt, magnesium salt and the like, ammonium salt), salts with organic base (e.g., organic amine salts such as triethylamine salt, diisopropylethylamine salt, pyridine salt, picoline salt, ethanolamine salt, triethanolamine salt, dicyclohexylamine salt, N,N'-dibenzylethylenediamine salt and the like), inorganic acid addition salt (e.g., hydrochloride, hydrobromide, sulfate, phosphate etc.), organic carboxylic acid or sulfonic acid addition salts (e.g., formate, acetate, trifluoroacetate, maleate, tartrate, fumarate, methanesulfonate, benzenesulfonate, toluenesulfonate etc.), salts with basic or acidic amino acid (e.g., arginine, aspartic acid, glutamic acid etc.) and the like, salts or acid addition salts with base can be mentioned.

In the present invention, in vivo and in vitro generally mean as used in the pertinent field. That is, "*in vivo*" refers to a state where an object biological function or reaction is expressed in the living organism, and "*in vitro*" refers to an expression of such function or reaction in a test tube (tissue culture system, cell culture system, cell free system etc.).

A method of obtaining a gene capable of becoming an index for predicting an anti-tumor effect of a histone deacetylase inhibitor of the present invention comprises at least the following steps:
(I) a step of examining an in vivo antitumor effect of a histone deacetylase inhibitor for tumor cells, and dividing the tumor cells into a cell type sensitive to the histone deacetylase inhibitor (sensitive tumor cell) and a cell type resistant thereto (resistant tumor cell), and
(II) a step of examining the gene expression pattern of each of the sensitive tumor cell and the resistant tumor cell divided in the above-mentioned step (I), and selecting
   (i) a gene showing high expression in the sensitive tumor cell and low expression in the resistant tumor cell, or
   (ii) a gene showing low expression in the sensitive tumor cell and high expression in the resistant tumor cell.

While the tumor cell as a test target to be used in the present invention (hereinafter simply referred to as a test cell) is not particularly limited as long as it has histone deacetylase, since evaluation of an antitumor effect of the histone deacetylase inhibitor, particularly tumor site specificity of the inhibitor, is one of the problems of the present invention, the test cell to be used is preferably derived from a tumor on which the effect is desired to be examined. For example, when the effect on prostate cancer is to be evaluated, PC-3 cell, which is a cultured human prostate cancer cell, and the like are used, and when the effect on kidney cancer is to be evaluated, ACHN cell, which is a cultured human kidney cancer cell and the like are used. Various cultured human cancer cells to be used as test cells including these cancer cells are commercially available, or available from various cell banks and the like. For examination of a long-term treatment effect, or effectiveness for individual patients, namely, tailor made medicine, it is possible to culture a cancer cell that can be obtained from a tumor of patient and use the cancer cell as a test cell.

The step of examining an in vivo antitumor effect of a histone deacetylase inhibitor in a tumor cell is conducted by, for example, the following procedures.

First, a tumor cell to be the test target is implanted into a test animal such as mouse, rat, rabbit, hamster, guinea pig and the like to prepare a system to experimentally form a tumor. In this system, a histone deacetylase inhibitor is administered after tumor implantation and the tumor growth rate is measured to evaluate an antitumor effect of the histone deacetylase inhibitor. The growth rate is conveniently determined by measuring the size of the tumor formed.

A tumor cell can be implanted to a test animal by a conventional method in this field. For example, a method comprising implanting, in a mouse, a part of a solid tumor subcultured and grown in a nude mouse can be mentioned. In addition, a histone deacetylase inhibitor can be administered in a similar amount and in a similar manner as in the administration generally expecting its antitumor effect. That is, application by intraperitoneal, intravenous, intramuscular or oral administration can be mentioned. While the dose varies depending on the kind and body weight of the test animal to be subjected to the administration, and the kind of the implanted cancer, about 1 mg/kg - 5 mg/kg is administered for general intravenous administration to a mouse.

The solvent to give a solution of a histone deacetylase inhibitor for the administration to a test animal is not particularly limited as long as it can dissolve the histone deacetylase inhibitor and it does not show toxicity to the tumor cell or the test animal as the test target. Generally, a concentrated solution is prepared with ethanol, PEG400, 10% HCO-60 solution, dimethyl sulfoxide and the like, a mixed solvent thereof and the like, and diluted to a desired concentration with physiological buffer such as physiological saline and the like and used.

When a given histone deacetylase inhibitor is administered in a certain dose, a tumor cell that exhibited a strong growth suppressing effect as shown by the growth suppressing rate of not less than 70% (preferably not less than 80%) is divided as a "sensitive tumor cell" to the histone deacetylase inhibitor, a tumor cell that exhibited a weak growth suppressing effect as shown by the growth suppressing rate of not more than 30% (preferably not more than 20%) is divided as a "resistant tumor cell" to the histone deacetylase inhibitor.

For example, when the histone deacetylase inhibitor is compound A, the cells are divided into a compound A sensitive tumor cell and a compound A resistant sensitive cell.

The compound A sensitive tumor cell is a tumor cell of the type exhibiting a strong growth suppressing effect by compound A on the tumor cell according to the aforementioned criteria, and, for example, prostate cancer cell PC-3 can be mentioned as shown in the below-mentioned Example. In addition, SC-6, which is a stomach cancer cell, is one kind of compound A sensitive tumor cells. On the other hand, compound A resistant tumor cell is a tumor cell of the type exhibiting only a weak growth suppressing effect by compound A according to the aforementioned criteria, and, for example, kidney cancer cell ACHN can be mentioned as shown in the below-mentioned Examples. In addition, A498, which is a kidney cancer cell, is one kind of compound A resistant tumor cells.

The step of examining the gene expression pattern in each of the divided sensitive tumor cell and the resistant tumor cell, and further selecting (i) a gene showing high expression in a sensitive tumor cell and low expression in a resistant tumor cell, or (ii) a gene showing low expression in a sensitive tumor cell and high expression in a resistant tumor cell (hereinafter the genes are to be also generally referred to as an antitumor effect predicting gene) can be conducted using the respective techniques described in the present specification and methods generally employed in this field. Preferably, a technique using a gene chip is employed in view of the advantage that a large number of gene expression patterns can be analyzed at once.

Analysis of the expression pattern of an antitumor effect predicting gene in a tumor cell can be an effective means for predicting the efficacy of a histone deacetylase inhibitor by an in vitro test without administration of the histone deacetylase inhibitor to patients.

The present invention provides a method of predicting an antitumor effect of a histone deacetylase inhibitor, which comprises examining the expression amount of an antitumor effect predicting gene in at least one kind, preferably two or more kinds of tumor cells.

The method of analyzing the gene expression pattern can be also performed using methods generally employed in this field.

For example, procedures described in the following can be mentioned.
(1) A gene, particularly mRNA, or protein is extracted from a test target tumor cell.
   This step can be also performed according to the methods generally employed in this field, and a kit containing a necessary reagent and the like can be also used. A gene or protein from a tumor cell can be extracted suitably from a confluent tumor cell. However, it is preferable to extract the gene or protein from a tumor fragment subcultured in an animal such as mouse and the like, implanted and grown, since a larger amount can be extracted.
(2) Using a substance having a specific affinity for a specific gene (or specific protein), the existence of a specific gene (or specific protein) is detected. Here, the specific gene (or specific protein) means a gene capable of becoming an index of efficacy prediction of the above-mentioned histone deacetylase inhibitor or a protein encoded by the gene, which is specifically the genes indicated in the below-mentioned Tables 1 - 3.

For a specific gene (or specific protein) to be measured in the present invention, one kind of measurement is sufficiently useful, but when a more detailed antitumor effect needs to be known, two or more kinds of specific genes (or specific proteins) are preferably measured simultaneously. More preferably, the expression of at least one kind of gene from each of a group of the genes showing high expression in a sensitive tumor cell and low expression in a resistant tumor cell and a group of the genes showing low expression in a sensitive tumor cell and high expression in a resistant tumor cell is preferably analyzed.

A substance having specific affinity for the specific gene or specific protein is free of any particular limitation as long as it has such a sensitivity as allows detection of expression in the test cells. As used herein, by the "specific affinity" is meant a property to hybridize or bind solely to an object gene or protein. As the substance to detect the specific gene, a substance absolutely complement to the whole or a part of said gene, or a substance containing one to several mismatches within the extent satisfying the above-mentioned property can be mentioned. Specifically, oligo- or poly-nucleotide containing a part or the entirety of the base sequence of the gene and complementary sequences thereof, and the like can be mentioned, and an appropriate substance is selected depending on the form of the gene to be detected. The derivation of the substance is not particularly limited as long as it has specific affinity for the gene, and it may be synthesized or formed by cleaving a necessary part from the gene and purifying the part by a conventional method. The substance may be labeled with a fluorescent substance, an enzyme, a radioisotope and the like. As the substance to be used for detecting a specific protein, for example, an antibody having specific affinity for the protein or a fragment thereof can be mentioned. The specific affinity thereof means an ability to specifically recognize the protein by an antigen-antibody reaction and bind thereto. The antibody and the fragment thereof are not particularly limited as long as they can specifically bind to the protein, and may be any of a polyclonal antibody, a monoclonal antibody and functional fragments thereof. These antibodies and functional fragments thereof can be produced according to a method generally employed in the pertinent field. These antibodies and fragments thereof may be labeled with a fluorescent substance, an enzyme, a radioisotope and the like.

Extraction of a gene, particularly mRNA, as well as extraction of a protein from the test cell can be performed according to a method generally employed in the pertinent field, or by an appropriate combination of such methods. When mRNA was extracted, its expression pattern is examined according to a method generally employed in the pertinent field, such as Northern blot, RT-PCR and the like, using a substance having specific affinity for the above-mentioned specific gene. On the other hand, when a protein was extracted, its expression pattern is examined according to a method generally employed in the pertinent field, such as immunoblot, Western blot and the like, using a substance (antibody, a fragment thereof etc.) having specific affinity for the above-mentioned specific protein.

Moreover, for the analysis of the expression pattern of the gene, the aforementioned methods as well as a technique using a gene chip is preferably employed in view of the advantage that a large number of gene expression patterns can be analyzed at once.

By the analysis of the expression pattern of an antitumor effect predicting gene, whether or not the tested histone deacetylase inhibitor effectively shows an antitumor activity against the tested tumor cell can be predicted. For example, when the genes are divided into a gene group showing twice or more higher expression in a sensitive tumor cell than in a resistant tumor cell (antitumor effect predicting gene A) and a gene group showing twice or more higher expression in a resistant tumor cell than in a sensitive tumor cell (antitumor effect predicting gene B), an antitumor effect of the histone deacetylase inhibitor can be expected in a tumor cell where the antitumor effect predicting gene A shows high expression, and/or a tumor cell where the antitumor effect predicting gene B shows low expression.

When a gene chip is used, the efficacy can be predicted by the following operations.
1. A part of the tumor tissue is removed from a cancer patient before administration of compound A.
2. RNA is extracted from the tumor tissue.
3. cDNA is synthesized, cRNA is synthesized, and cRNA is fragmented from the extracted RNA.
4. Hybridization is performed using a gene chip.
5. The gene chip is washed, stained and scanned.
6. Homology with the expression pattern^{*)} of the efficacy predicting candidate gene group in the sensitive tumor is examined (^{*)} a gene showing high expression amount (normalized data of not less than 1) is shown in red and a gene showing low expression amount (normalized data of less than 1) is shown in blue on an analysis screen).
7. A tumor having higher homology is evaluated as having higher possibility of efficacy expression.

When a clinically removed tumor cell from a patient is used as a test cell, prediction of an antitumor effect reflecting individual specificity of the individual patient can be achieved.

The present invention can provide a screening method of a histone deacetylase inhibitor having an antitumor activity specific for a tumor site (kind), by utilizing the aforementioned evaluation methods of the antitumor effect of a histone deacetylase inhibitor. The tumor site specificity of individual inhibitor can be evaluated using a test cell derived from the target tumor, treating with a histone deacetylase inhibitor to be examined for the effect, and examining the presence of the antitumor effect according to the aforementioned methods.

### Examples

The present invention is explained specifically and in detail in the following by referring to Examples, which are not to be construed as limitative.

### Example 1

### Evaluation of compound A sensitive tumor and compound A resistant tumor

### (1) Preparation of pharmaceutical agent

A necessary amount of FK228 was weighed and a solvent (10% HCO-60/saline) was added. The mixture was sonicated to allow for dissolution. A positive control substance Paclitaxel was dissolved in Cremophor EL/ethanol (1:1) solution to 24 mg/mL prior to the testing, and preserved in a refrigerator. When in use, it was diluted with a 9-fold amount of physiological saline to 2.4 mg/mL (solvent component: 5% Cremophor EL-5% ethanol-90% saline).

### (2) Test animal

For antitumor test of the pharmaceutical agent, BALB/cANnNCrj-nu/nu mice (male, 6-week-old) were purchased from Charles River Japan and, after acclimation for not less than one week, used for the test. The mice were reared under an SPF environment and allowed a free access to water and feed.

### (3) Test tumor

Cultured human kidney cancer cell line 1 (ACHN: available from ATCC) and human culture prostate cancer cell line 1 (PC-3: available from ATCC) were subcutaneously implanted at 2-3×10⁷ cells in a nude mouse. A grown solid tumor was subcultured for not less than 3 generations and used for the test.

### (4) Experimental implantation and grouping

A solid tumor subcultured in a nude mouse was subcutaneously implanted in the right back of a mouse as an about 3 mm square tumor tissue fragment. After the tumor implantation, when the tumor volume (1/2×longer diameter×shorter diameter²) reached 100-300 mm³, the mice were grouped into 6 mice per group to level the tumor size.

### (5) Administration

The administration was started on the day of grouping (Day 0). FK228 was intravenously administered to an FK228 administration group 3 times every 4 days (q4dx3) (3.2 and 1.8 mg/kg). Paclitaxel (24 mg/kg) was intravenously administered for 5 consecutive days (qd×5) to a positive control substance Paclitaxel administration group. Only a solvent (10% HCO-60/saline) was administered (q4dx3) to a control group. The amount of liquid for each administration was calculated (0.1 mL/10 g body weight) based on the body weight measured on the administration day. Note that 3.2 mg/kg/day (q4dx3) of FK228 and 24 mg/kg/day (qdx5) of Paclitaxel were the maximum tolerated doses (MTD) thereof.

### (6) Measurement of tumor size and body weight

The tumor size (longer diameter, shorter diameter) and body weight were measured twice a week from Day 0. (7) Evaluation of antitumor effect

The level of tumor growth was evaluated based on the tumor growth rate (Relative Tumor Volume). The growth suppression rate was expressed in a relative proportion of tumor volume after day 0 to the tumor volume at Day 0 as 1.

The results are shown in Fig. 1. FK228 showed a strong antitumor action on PC-3 at a dose of 3.2 mg/kg (Fig. 1(a)) but did not show an antitumor action on ACHN (Fig. 1(b)).

### Example 2

### Evaluation of compound A sensitive tumor and compound A resistant tumor

### <material, procedure>

### (1) test material

drug: compound A (FK228)
   dose: 3.2 mg/kg
   administration volume: 10 mL/kg
   solvent: 10% HCO-60/saline solution
   dosage form: solution (prepared when in use)
tumor cell: human prostate cancer PC-3 (tumor fragment 3 mmx3 mmx3 mm/mouse implantation site s.c.)
   human gastric cancer SC-6; obtained from Central Institute for Experimental Animals (tumor fragment 3 mmx3 mmx3 mm/mouse implantation site s.c.)
   human kidney cancer ACHN (tumor fragment 3 mmx3 mmx3 mm/mouse implantation site s.c.)
   human kidney cancer A498; obtained from ATCC (tumor fragment 3 mm×3 mmx3 mm/mouse implantation site s.c.) Subcultured animal: male BALB c/nu/nu

### (2) procedure and results

By the method shown in Example 1, 3 mm square tumor fragments (human prostate cancer PC-3, human stomach cancer SC-6, human kidney cancer ACHN and human kidney cancer A498) were subcutaneously implanted in nude mice, and when the tumor volume (long diameter 9 mm, short diameter 8 mm) reached about 100-300 mm³, FK228 (3.2 mg/kg) was intravenously administered. The tumor growth suppressing rates then were 98%, 84%, 20% and 29%, respectively. From these results, PC-3 and SC-6 were evaluated as compound A sensitive tumors and ACHN and A498 were evaluated as compound A resistant tumors.

### Example 3

### Analysis of gene expression pattern of compound A sensitive tumor and compound A resistant tumor using gene chip

The gene expression pattern of the tumor for which sensitivity and resistivity were confirmed in Example 2 was examined.

### (1) test material

### tumor cell human prostate cancer PC-3 (tumor fragment 3 mmx3 mmx3 mm/mouse implantation site s.c.)

human gastric cancer SC-6; obtained from Central Institute for Experimental Animals (tumor fragment 3 mmx3 mmx3 mm/mouse implantation site s.c.)
human kidney cancer ACHN (tumor fragment 3 mmx3 mmx3 mm/mouse implantation site s.c.)
human kidney cancer A498; obtained from ATCC (tumor fragment 3 mmx3 mmx3 mm/mouse implantation site s.c.) Subcultured animal: male BALB c/nu/nu

- RNA extraction:: RNeasy Mini Kit (50) (Qiagen)
RNase, DNase free water (Life Technologies)
- DNA synthesis:: Superscript Choice System (Life Technologies)
Ethachinmate (Nippon gene)
T7-(dT)24 Primer (Amersham Pharmacia)
- cRNA synthesis:: BioArray RNA Transcript Labeling Kit (Amersham Pharmacia)
- cRNA fragmentation:: Trizma Base (SIGMA)
glacial acetic acid (SIGMA)
magnesium acetate (SIGMA)
potassium acetate (SIGMA)
- Hybridization:: Eukaryotic Hybridization Control Kit (Amersham Pharmacia)
0.5 M EDTA solution (SIGMA)
MES Sodium Salt (SIGMA)
MES Free Acid Monohydrate (SIGMA)
Herring Sperm DNA (Promega)
Acetylated Bovine Serum Albumin Soln. (Life Technologies)
- Chip used:: HuGeneFL array (Amersham Pharmacia)

### (2) Cell preparation and RNA extraction

A 3 mm square tumor fragment (human prostate cancer PC-3, human stomach cancer SC-6, human kidney cancer ACHN and human kidney cancer A498) was subcutaneously implanted in a nude mouse and, when the tumor reached about 100 to 300 mm³ (longer diameter 9 mm, shorter diameter 8 mm), the tumor was removed and total RNA was extracted according to the protocol of RNeasy Mini Kit (50) (Qiagen). RNA was quantified and confirmed by electrophoresis.

### (3) Synthesis of cRNA

According to the GeneChip manual, Chapter 2 - Chapter 4, and the manuals of RNeasy Mini Kit and RNA Transcript Labeling Kit, RNA was purified, cDNA was synthesized, cRNA was synthesized and cRNA was fragmented.

### (4) Hybridization, Washing-staining, Scanning

Hybridization, washing-staining and scanning were conducted according to the GeneChip manual Chapter 5 - Chapter 7.

### (5) Analysis

Analyzed using GeneSpring (microarray data analysis soft: manufactured by Silicon Genetics).

The analysis conditions were as follows.

### Analysis conditions

· Raw data are not less than 100 in at least one of four tumor cells.
· Difference in normalized data between compound A sensitive tumors, or compound A resistant tumors is not more than 2-fold.
· Difference in normalized data between compound A sensitive tumors, or compound A resistant tumors is not less than 2-fold.

As a result, 27 genes showing twice or more higher expression in a compound A sensitive tumor than in a compound A resistant tumor were obtained (Table 1), and 49 genes showing twice or more higher expression in a compound A resistant tumor than in a compound A sensitive tumor were obtained (Tables 2 - 3).

**Table 1:**

| genes that showed high expression in a compound A sensitive tumor and low expression in a compound A resistant tumor | | |
|---|---|---|
| | Gene Accession | No. Description |
| 1 | L11005_at | aldehyde oxidase 1 |
| 2 | X12517_at | small nuclear ribonucleoprotein polypeptide C |
| 3 | U43944_at | malic enzyme 1, soluble |
| 4 | U52100_at | epithelial membrane protein 2 |
| 5 | U65579_at | NADH dehydrogenase (ubiquinone) Fe-S protein 8 (23kD) (NADH-coenzyme Q reductase) |
| 6 | X06272_at | signal recognition particle receptor ('docking protein') |
| 7 | D42047_at | The ha3662 gene product is related to mouse glycerophosphate dehydrogenase. |
| 8 | D90086_at | pyruvate dehydrogenase (lipoamide) beta |
| 9 | U61734_s_at | |
| 10 | J03798_at | small nuclear riboprotein Sm-D |
| 11 | X05299_at | centromere protein B (80kD) |
| 12 | L34155_at | laminin, alpha 3(nicein (150kD), kalinin (165kD), BM600 (150kD), epilegrin) |
| 13 | U60521_at | caspase 9, apoptosis-related cysteine protease |
| 14 | Y10807_s_at | HMT1 (hnRNP methyltransferase, S. cerevisiae)-like 2 |
| 15 | U44754_at | small nuclear RNA activating complex, polypeptide 1, 43kD |
| 16 | U78107_at | N-ethylmaleimide-sensitive factor attachment protein, gamma |
| 17 | U60644_at | similar to Vaccinia virus HindIII K4L 0RF, and to Vaccinia virus p37 (HindIII F13L 0RF) |
| 18 | U32986_s_at | damage-specific DNA binding protein 1 (127kD) |
| 19 | D59253_at | RNA-binding protein that has two RNP consensus motifs |
| 20 | U41767_s_at | a disintegrin and metalloproteinase domain 15 (metargidin) |
| 21 | U10550_at | Source: Human Gem GTPase (gem) mRNA, complete cds. |
| 22 | D38521_at | The ha0919 gene product is novel. |
| 23 | J04823_rnal_at | cytochrome c oxidase subunit VIII |
| 24 | M21154_at | S-adenosylmethionine decarboxylase 1 |
| 25 | X78565_at | hexabrachion (tenascin C, cytotactin) |
| 26 | D50405_at | histone deacetylase 1 |
| 27 | X04366_at | calpain, large polypeptide L1 |

Table 2 - 3: genes that showed low expression in a compound A sensitive tumor and high expression in a compound A resistant tumor

**Table 2:**

| | Gene Accession No. | Description |
|---|---|---|
| 1 | U84487_at | small inducible cytokine subfamily D (Cys-X3-Cys), member 1 (fractalkine, neurotactin). |
| 2 | M33600_f_at | major histocompatibility complex, class II, DR beta 5 |
| 3 | J03474_at | amyloid A precursor |
| 4 | L24564_at | Ras-related associated with diabetes |
| 5 | J05428_at | UDP glycosyltransferase 2 family, polypeptide B7 |
| 6 | L03840_s_at | fibroblast growth factor receptor 4 |
| 7 | M35878_at | insulin-like growth factor binding protein 3 |
| 8 | HG4318-HT4588_s_at | |
| 9 | X87241_at | FAT tumor suppressor (Drosophila) homolog |
| 10 | M59807_at | natural killer cell transcript 4 |
| 11 | HG2379-HT3996_s_at | |
| 12 | D16532_at | very low density lipoprotein receptor |
| 13 | U37546_s_at | apoptosis inhibitor 1 |
| 14 | X72889_at | Source: H.sapiens hbrm mRNA. |
| 15 | U66838_at | cyclin Al |
| 16 | Z23090_at | heat shock 27kD protein 1 |
| 17 | U16031_at | signal transducer and activator of transcription 6, interleukin-4 induced |
| 18 | D84110_at | alternative splicing (see also D84107-D84111) |
| 19 | M55998_s_at | |
| 20 | D28124_at | neuroblastoma candidate region, suppression of tumorigenicity 1 |
| 21 | HG3548-HT3749_at | |
| 22 | X86809_at | phosphoprotein enriched in astrocytes 15 |
| 23 | M79462_at | promyelocytic leukemia |
| 24 | M59465_at | tumor necrosis factor, alpha-induced protein 1 (endothelial) |
| 25 | HG2379-HT3997_s_at | |
| 26 | Z36715_at | ELK3, ETS-domain protein (SRF accessory protein 2) NOTE: Symbol and name provisional. |
| 27 | U41654_at | putative GTP-binding protein, homolog of small GTPase family members |
| 28 | U72882_s_at | Source: Human interferon-induced leucine zipper protein (IFP35) mRNA, partial cds. |

**Table 3:**

| | | |
|---|---|---|
| 29 | U72649_at | rat PC3 and murine TIS21 genes homolog |
| 30 | U47621_at | |
| 31 | L22214_at | adenosine A1 receptor |
| 32 | U68494_at | Source: Human hbc647 mRNA sequence. |
| 33 | X69699_at | paired box gene 8 |
| 34 | X63717_at | tumor necrosis factor receptor superfamily, member 6 |
| 35 | U04285_s_at | |
| 36 | L38969_at | Source: Homo sapiens thrombospondin 3 (THBS3) mRNA, complete cds. |
| 37 | Y10032_at | Source: H.sapiens mRNA for putative serine/threonine protein kinase. |
| 38 | L48513_at | paraoxonase 2 |
| 39 | U89942_at | lysyl oxidase-like 2 |
| 40 | Z12173_at | glucosamine (N-acetyl)-6-sulfatase (Sanfilippo disease IIID) |
| 41 | U59423_at | Sma and Mad homolog |
| 42 | X68277_at | dual specificity phosphatase 1 |
| 43 | U45878_s_at | HIAP-1 |
| 44 | L08187_at | |
| 45 | X71874_cds1_at | proteasome (prosome, macropain) subunit, beta type, 10 |
| 46 | D50863_at | testis-specific kinase 1 |
| 47 | X91911_s_at | |
| 48 | U65011_at | encodes tumor antigen recognized by cytolytic T lymphocytes |
| 49 | U90313_at | glutathione-S-transferase like |

### Industrial Applicability

The gene obtained by the method of the present invention, which can be an index of the efficacy, particularly an antitumor effect, of a histone deacetylase inhibitor is suggested to be correlated to the efficacy of histone deacetylase inhibitor, as well as to the sensitivity or resistivity to the histone deacetylase inhibitor, and the possibility of use of the gene as an efficacy predicting marker of a histone deacetylase inhibitor has been shown.

### Sequence Listing Free Text

SEQ ID; No 1: Xaa is an amino acid represented by the formula

   NH₂C(CHCH₃)COOH.

The carboxyl group of the formula COOHCH₂CH(CHCHC₂H₄SH)OH is bonded with an amino group of Val, which is the first amino acid, a hydroxyl group is bonded with a carboxyl group of Val, which is the fourth amino acid, and an SH group is disulfide-bonded with an SH group of Cys, which is the second amino acid.

This application is based on a patent application No. 2003-041790 filed in Japan, the contents of which are hereby incorporated by reference.

## Claims

1. A method of obtaining a gene capable of becoming an index for predicting efficacy of a histone deacetylase inhibitor, which comprises at least
(I) a step of examining an in vivo antitumor effect of a histone deacetylase inhibitor for tumor cells, and dividing the tumor cells into a cell type sensitive to the histone deacetylase inhibitor (sensitive tumor cell) and a cell type resistant thereto (resistant tumor cell), and
(I) a step of examining the gene expression pattern of each of the sensitive tumor cell and the resistant tumor cell divided in the above-mentioned step (II), and selecting
(i) a gene showing high expression in the sensitive tumor cell and low expression in the resistant tumor cell, or
(ii) a gene showing low expression in the sensitive tumor cell and high expression in the resistant tumor cell.

2. The method of claim 1, wherein the histone deacetylase inhibitor is a compound represented by the following chemical formula (I): 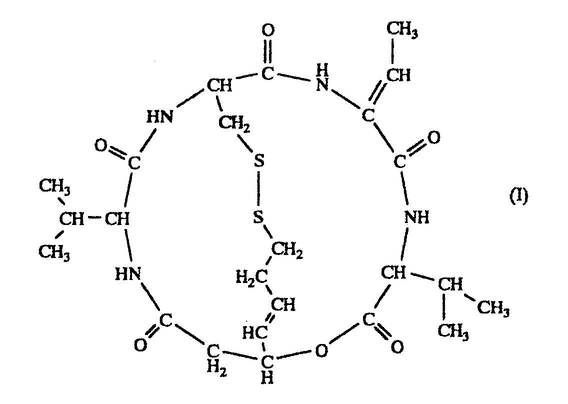 or a pharmaceutically acceptable salt thereof.

3. The method of claim 1 or 2, wherein the tumor cell is derived from prostate cancer, stomach cancer or kidney cancer.

4. A method of predicting an antitumor effect of a histone deacetylase inhibitor, which comprises examining an expression amount of at least one gene obtained by the method of claim 1 in at least one kind of tumor cell.

5. The method of claim 4, wherein the histone deacetylase inhibitor is a compound represented by the following chemical formula (I): 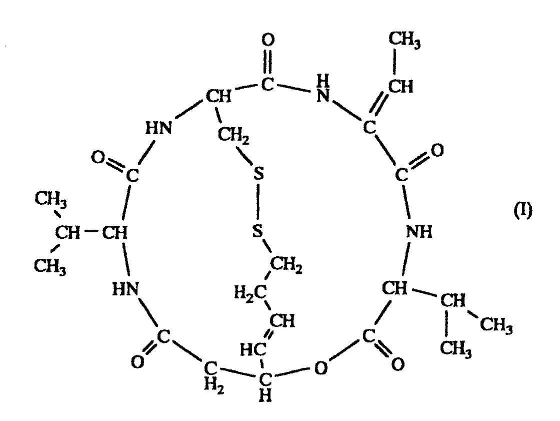 or a pharmaceutically acceptable salt thereof.

6. The method of claim 4 or 5, wherein the at least one gene shows high expression in a sensitive cell and low expression in a resistant tumor cell.

7. The method of claim 4 or 5, wherein the at least one gene shows low expression in a sensitive cell and high expression in a resistant tumor cell.

8. A method of predicting an antitumor effect of a histone deacetylase inhibitor, which comprises examining an expression amount of at least one gene showing high expression in a sensitive cell and low expression in a resistant tumor cell, and at least one gene showing low expression in a sensitive cell and high expression in a resistant tumor cell, which are obtained by the method described in claim 1, in at least one kind of tumor cell.

9. The method of any of claims 4 to 8, wherein the tumor cell is derived from prostate cancer, stomach cancer or kidney cancer.


SEQUENCE LISTING
<110> FUJISAWA PHARMACEUTICAL CO., LTD.
<120> Prediction-method for anti-tumor effect of histone deacetylase
inhibitor
<130> 09608
<150> JP 2003-041790
<151> 2003-02-19
<160> 1
<170> PatentIn version 3.1
<210> 1
<211> 4
<212> PRT
<213> Chromobacterium sp.
<220>
<221> SITE
<222> (3)
<223> Xaa is an amino acid represented by the formula NH2C(CHCH3)COOH.
<221> SITE
<222> (1),(2),(4)
<223> In the formula COOHCH2CH(CHCHC2H4SH)OH, the carboxylic group is bonded
with the amino group of the first amino acid Val, the hydroxyl group is bonded
with the carboxylic group of the fourth amino acid Val, and the SH group is
bonded with the SH group of the second amino acid Cys via a disulfide bond.
<400> 1
Val Cys Xaa Val 4